# EUROPÄISCHE PATENTSCHRIFT

(11) **EP 0 742 205 B1**
(45) Veröffentlichungstag und Bekanntmachung des Hinweises auf die Patenterteilung: **23.08.2000**
(21) Anmeldenummer: 96106948.1
(22) Anmeldetag: 03.05.1996
(51) Int. Cl.: C07C 403/24

(54) **Verfahren zur Herstellung von beta-Carotin-Präparaten mit hohem 9(Z)-Gehalt**
Process for the preparation of beta-carotene with high 9(Z) content
Procédé pour la préparation de compositions de bêta-carotène à haute teneur en 9(Z)

(30) Priorität: 12.05.1995 DE 19517422
(43) Veröffentlichungstag der Anmeldung: 13.11.1996
(73) Patentinhaber: BASF AKTIENGESELLSCHAFT, 67056 Ludwigshafen (DE)
(72) Erfinder: Paust, Joachim, Dr., 67141 Neuhofen (DE); John, Michael, Dr., 67245 Lambsheim (DE)

(56) Entgegenhaltungen:
- EP-A- 0 659 739
- DE-C- 954 247
- FR-A- 2 367 771
- US-A- 3 466 335
- PURE APPL. CHEM. (PACHAS,00334545);91; VOL.63 (1); PP.35-44, F. HOFFMANN-LA ROCHE LTD.;DEP. VITAMIN NUTR. RES.; BASEL; CH-4002; SWITZ. (CH), XP000607048 BERNHARD K ET AL: "Recent advances in the synthesis of achiral carotenoids"

## Beschreibung

Die Erfindung betrifft ein Verfahren zur Herstellung von β-Carotin-Präparaten mit hohem Anteil an dem 9(Z)-Isomeren, ausgehend von Mutterlaugen der technischen Herstellung von β-Ionylidenethyltriarylphosphoniumsalzen (C₁₅-Triarylphosphoniumsalzen) durch Wittig-Reaktion eines an dem 9Z-Isomeren angereicherten C₁₅-Triarylphosphoniumsalzes direkt mit β-Apo-12'-carotinal oder aber mit 2,7-Dimethyl-2,4,6-octatrien-1,8-dial und anschließend mit dem (E)-konfigurierten C₁₅-Triarylphosphoniumsalz.

Aus DE-C-95 42 47 ist ganz allgemein die Herstellung von β-Carotin durch Wittig-Reaktion von einem 3-Ionylidenethyltriphenylphosphoniumhalogenid mit ½ Mol 2,7-Dimethyl-2,4,6-octatrien-1,8-dial und anschließende Abtrennung des als Nebenprodukt gebildeten Triphenylphosphinoxids bekannt.

Aus Pure & Appl. Chem. Vol. 63 (1991), No. 1, Seiten 35-44, bes. 41-42, ist die Herstellung von (9Z)-β-Carotin durch Wittig-Reaktion von (9Z)-Retinal mit Retinyltriphenylphosphoniumchlorid und anschließende Reinigung des erhaltenen Gemisches aus (9Z)- und (9Z,15Z)-β-Carotin durch Säulenchromatographie und Kristallisation bekannt. Das (9Z)-Retinal wurde dabei vorher durch Isomerisierung von (all-E)-Retinol oder seinem Acetat und anschließende Oxidation des erhaltenen (9Z)-Retinols gewonnen. Nachteilig an diesem Verfahren ist, daß es für eine Anwendung im industriellen Maßstab zu aufwendig ist.

9(Z)-β-Carotin der Formel I kommt in der Natur vor. Gemäß US 5,310,554 bilden Dunaliella-Algen bei intensiver Sonneneinstrahlung teilweise mehr 9(Z)-β-Carotin als all-(E)-β-Carotin. Dem 9(Z)-β-Carotin werden gemäß US 5,310,554 Vorteile bezüglich der Formulierung und der Bioverfügbarkeit gegenüber der all-(E)-Form zugeschrieben. Außerdem ist 9(Z)-β-Carotin gemäß Angaben in Nature Vol. 355 (1992) Seiten 359-61 und Archives of Biochemistry and Biophysics, Vol. 313 (1994) Seiten 150-55 als Vorstufe für 9(Z)-Retinsäure anzusehen, die den RXRa-Zellrezeptor aktiviert und damit die embryonale Entwicklung sowie Differenzierung und Proliferation von Zellen steuert.

Die Isolierung von 9(Z)-β-Carotin enthaltenden Präparaten aus biologischem Material ist sehr aufwendig und schließt die Abtrennung anderer lipophiler Substanzen ein. Es bestand daher die Aufgabe, entsprechende Präparate auf möglichst einfach Weise auf chemischem Wege herzustellen.

Bei der Herstellung des für technische Vitamin-A-Synthesen sowie für die Herstellung von anderen Vitamin-A-Abkömmlingen, wie Retinal und der Retinsäure, benötigten C₁₅-Triarylphosphoniumsalzes (siehe z.B. H. Pommer et al. in Angew. Chem. 77 (1965) 277-360) fällt nach Abtrennung des Wertproduktes eine Mutterlauge an, in der neben all-(E)-C₁₅-Triphenylphosphoniumsalz das 9(Z)-Isomere in einem Anteil von 10 bis 60 Gew.-%, insbesondere 30 bis 40 Gew.-%, bezogen auf Gesamt-C₁₅-Triarylphosphoniumsalz, enthalten ist. Eine direkte Verwendung dieser Mutterlauge für die Herstellung von β-Carotinpräparaten mit einem hohen Gehalt an (Z)-Isomeren ist nicht ohne Schwierigkeiten möglich.

Es war die Aufgabe der Erfindung, ein Verfahren zu entwickeln, mit dessen Hilfe die gewünschten β-Carotinpräparate aus diesen Mutterlaugen der C₁₅-Triphenylphosphoniumsalzgewinnung bzw. ganz allgemein aus Mutterlaugen der C₁₅-Triarylphosphoniumsalzgewinnung hergestellt werden können.

Gegenstand der Erfindung ist ein Verfahren zur Herstellung von β-Carotinpräparate mit Anteilen von 60 bis 80 % (HPLC-Flächen-%) an 9(Z)-β-Carotin der Formel I 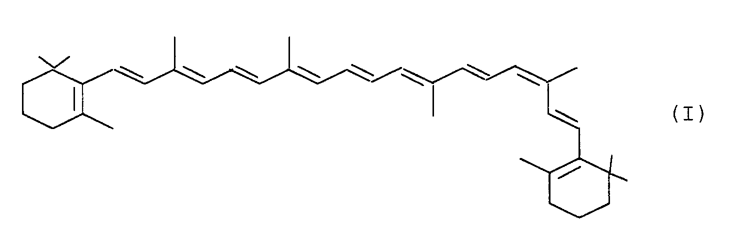 ausgehend von Mutterlaugen der technischen Herstellung von β-Ionylidenethyl-triarylphosphoniumsalzen (C₁₅-Triarylphosphoniumsalzen), das dadurch gekennzeichnet ist, daß man
A. in den aus den Mutterlaugen durch Extraktion mit Wasser und Einengen der wäßrigen Phase isolierten C₁₅-Triarylphosphoniumsalzen den Anteil an 9(Z)-C₁₅-Triarylphosphoniumsalzen dadurch anreichert, daß man das ölige C₁₅-Triarylphosphoniumsalzgemisch in einem niederen Alkanol löst und das beim Abkühlen auskristallisierende all-(E)-C₁₅-Triarylphosphoniumsalz abtrennt,
B. das erhaltene, an dem 9(Z)-Isomeren der allgemeinen Formel II 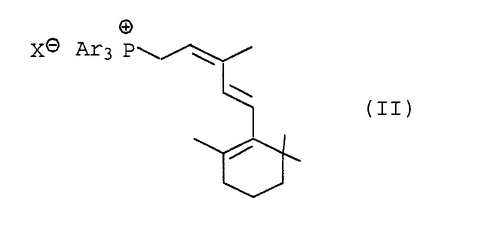 in der X für Halogen oder den Rest (HSO₄)⁻ und Ar für ggf. substituiertes Phenyl steht,
   angereicherte C₁₅-Triarylphosphoniumsalz in Gegenwart einer Base in einem für Wittig-Reaktionen üblichen Lösungsmittel
   a) entweder direkt mit all-(E)-β-Apo-12'-carotinal der Formel III 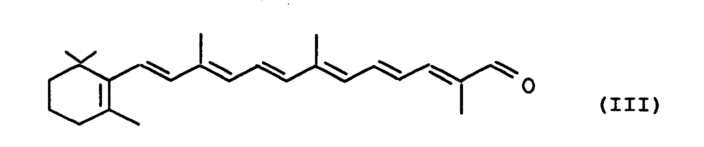 oder aber
   b) zunächst mit dem symmetrischen C₁₀-Dialdehyd der Formel IV 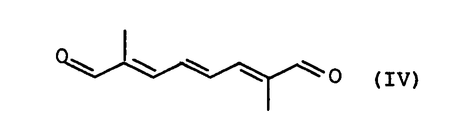 umsetzt und dann das dabei erhaltene 9(Z)-β-Apo-12'-carotinal der Formel V 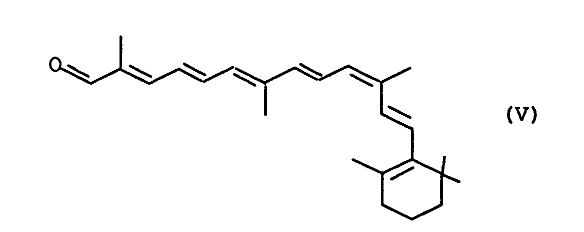 in Gegenwart einer Base in einem für Wittig-Reaktionen üblichen Lösungsmittel mit einem 9-(E)-β-Ionylidenethyl-triarylphosphoniumsalz der allgemeinen Formel VI 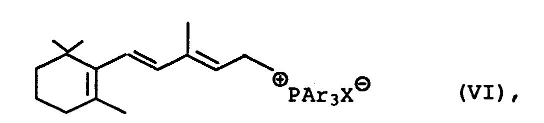 in der X^{⊖} und Ar die oben angegebenen Bedeutung haben, umsetzt und
C. das erhaltene und auf übliche Weise isolierte β-Carotin zwecks Isomerisierung der gebildeten (Z)-konfigurierten Doppelbindung in 11- bzw. 11'-Stellung von β-Carotin einer thermischen Isomerisierung unterwirft.

Mit besonderem Vorteil gelingt das erfindungsgemäße Verfahren, wenn man im Reaktionsschritt A den Anteil an dem 9-(Z)-C₁₅-Triarylphosphoniumsalz aus der Mutterlauge der C₁₅-Triarylphosphoniumsalz-Herstellung dadurch anreichert, daß man
a) die Mutterlauge mit Wasser versetzt und die organische Phase abtrennt,
b) die wäßrige Phase unter schonenden Bedingungen einengt und dabei einen Lösungsmittelaustausch von Wasser zu Isopropanol vornimmt und
c) das durch Abkühlen auskristallisierende all-(E)-C₁₅-Triarylphosphoniumsalz abtrennt.

Die Herstellung der C₁₅-Triarylphosphoniumsalze erfolgt in an sich bekannter Weise im allgemeinen in aromatischen Kohlenwasserstoffen, wie Toluol oder Xylol, in chlorierten Kohlenwasserstoffen, wie Methylenchlorid, oder in niederen Alkanolen, wie Methanol oder Isopropanol, in Acetonitril oder aber in Gemischen dieser Lösungsmittel.

Dementsprechend bestehen die für das erfindungsgemäße Verfahren verwendeten Mutterlaugen aus einem dieser Lösungsmittel oder einem Lösungsmittelgemisch, aus 9-(E)- und 9-(Z)-C₁₅-Triarylphosphoniumsalz und Nebenprodukten, wie Säurespuren, Reaktionswasser und C₁₅-Kohlenwasserstoff, aus Triphenylphosphinoxid, sowie Triarylphosphoniumsalzen der Halogenwasserstoffsäure bzw. Schwefelsäure.

Sie enthalten - je nach den Reaktionsbedingungen - das 9-(Z)-Isomere in einem Anteil von 10 bis 60 %, vorzugsweise 30 bis 40 %, bezogen auf die Gesamt-C₁₅-Triarylphosphoniumsalzmenge. Aus dieser Mutterlauge werden die C₁₅-Triarylphosphoniumsalze mit Wasser extrahiert und die wäßrige Phase unter schonenden Bedingungen eingeengt. Das so erhaltene Öl wird in soviel von einem niederen Alkanol, vorzugsweise in soviel Isopropanol, aufgenommen, daß eine Konzentration des Salzes von 30 bis 70 Gew.-%, vorzugsweise 40 bis 60 Gew.-%, insbesondere 45 bis 55 Gew.-% vorliegt. Anschließend läßt man die erhaltene Lösung bei Temperaturen von -50 bis 25°C, vorzugsweise -30 bis 0°C stehen, wobei all-(E)-C₁₅-Triarylphosphoniumsalz auskristallisiert und so abgetrennt werden kann. Nach dieser Anreicherung des 9(Z)-C₁₅-Salzes liegt in der Mutterlauge ein Verhältnis von 9(Z)- zu all-(E)-C₁₅-Triphenylphosphoniumsalz von etwa 1:1 bis zu 50:1 vor. Gemäß dem Ausführungsbeispiel wurde ein Verhältnis von etwa 8,4:1 erzielt.

Eine weitere Anreicherung des 9-(Z)-Isomeren ist auf Basis einer kinetischen Kontrolle der anschließenden Wittig-Reaktion möglich, da das verbliebene all-(E)-Isomere sehr viel schneller die Wittig-Reaktion eingeht als das 9(Z)-C₁₅-Triarylphosphoniumsalz. Wird nämlich das C15-Triarylphosphoniumsalz-Gemisch in einer Wittig-Reaktion mit einer dem noch enthaltenen Anteil an all-(E)-C₁₅-Salz entsprechenden Menge einer Aldehydverbindung, beispielsweise mit Acetaldehyd umgesetzt, so gestattet eine saure Aufarbeitung des Reaktionsansatzes die Isolierung von reinem 9(Z)-C₁₅-Triarylphosphoniumsalz aufgrund der kinetischen Differenzierung.

Die Verknüpfung des mit dem 9(Z)-Isomeren der allgemeinen Formel II angereicherten C₁₅-Triarylphosphoniumsalzes, mit dem all-(E)-β-Apo-12'-carotinal der Formel III gemäß Reaktionsstufe Ba) sowie dessen Verknüpfung mit dem C₁₀-Dialdehyd der Formel IV zu dem 9(Z)-β-Apo-12'-carotinal der Formel V gemäß Reaktionsstufe Bb) bzw. die weitere Verknüpfung des gemäß Stufe Bb) erhaltenen 9(Z)-β-Apo-12'-carotinals mit all-(E)-Triarylphosphoniumsalzen der allgemeinen Formel VI erfolgen im allgemeinen unter den in der Polyenchemie für Wittig-Reaktionen üblichen Bedingungen. Bezüglich näherer Details, über die allgemeinen Reaktionsbedingungen für Wittig-Reaktionen verweisen wir auf DE 10 68 703 oder DE 10 68 705.

Als Basen für die Wittig-Reaktion verwendet man im allgemeinen Alkalihydroxide, Erdalkalihydroxide, Amine, Ammoniak oder Alkalicarbonate.

Mit besonderem Vorteil verwendet man schwache Basen, wie organische Amine, Ammoniak, Alkalicarbonate oder Magnesiumhydroxid, aber auch Alkalialkoholate.

Die Umsetzung wird im allgemeinen bei Temperaturen von -20°C bis 50°C, vorzugsweise zwischen 0°C und 20°C durchgeführt.

Geeignete Lösungsmittel sind solche, die unter den Reaktionsbedingungen inert sind und die die Edukte in ausreichendem Maße lösen. Beispielsweise empfiehlt es sich, den schwerlöslichen Dialdehyd der Formel IV in Gegenwart von Methylenchlorid oder Dimethylformamid umzusetzen. Das Reaktionsmedium kann als homogene Phase vorliegen oder zweiphasig sein. So kann man beispielsweise in reinem Methylenchlorid, reinem Dimethylformamid, in niederen Alkanolen oder in Gemischen dieser Lösungsmittel arbeiten. Es ist aber auch möglich in zweiphasigen Systemen wie Methylenchlorid/Wasser oder Heptan/Wasser zu arbeiten. Zur Durchführung des erfindungsgemäßem Verfahrens kann man dabei entweder beide Ausgangsverbindungen in dem Lösungsmittel vorlegen und dazu die Base geben oder aber eine Lösung des C₁₅-Triarylphosphoniumsalzes vorlegen, die Base zufügen und erst danach eine Lösung des entsprechenden Aldehyds zufügen. Die Aufarbeitung der Ansätze erfolgt beispielsweise durch Aufnehmen des Wertproduktes in Heptan und Abtrennen von Triarylphosphinoxid mit Hilfe von Wasser-Methanol-Gemischen. Reste der eingesetzten β-Apo-12'-carotinale können gewünschtenfalls durch Filtrieren über Silikagel abgetrennt werden.

Besonders hohe Anteile an 9-(Z)-Isomeren erhält man bei dem erfindungsgemäßen Verfahren, wenn man das erhaltene und auf übliche Weise isolierte β-Carotin in Reaktionsstufe C. zwecks Isomerisierung der (Z)-konfigurierten Doppelbindung in 11- bzw. 11'-Stellung vom gebildeten β-Carotin in inerten Kohlenwasserstoffen oder Alkanolen, vorzugsweise in Heptan oder Isobutanol, etwa 1 bis 2 Stunden auf Temperaturen von 70 bis 80°C erwärmt, und/oder wenn man das in Reaktionsstufe Bb) zunächst erhaltene und auf übliche Weise isolierte rohe β-Apo-12'-carotinal der Formel V zwecks Isomerisierung der (Z)-konfigurierten Doppelbindung in 11-Stellung des als Nebenprodukt gebildeten 9(Z), 11(Z)-Isomeren in inerten Kohlenwasserstoffen oder Alkanolen, vorzugsweise in Heptan oder Isobutanol etwa 1 bis 2 Stunden auf Temperaturen von 70°C bis 80°C erwärmt.

Mit Hilfe des erfindungsgemäßen Verfahrens können auf relativ einfache Weise β-Carotin-Präparate mit 9(Z)-Anteilen von etwa 60 bis 80 % (HPLC-Flächen-%) erhalten werden. Etwa 10 % entfallen auf all-(E)-β-Carotin, der Rest auf andere (Z)-Verbindungen und Isomere mit zwei (Z)-konfigurierten Doppelbindungen.

Die folgenden Ausführungsbeispiele sollen die Durchführung des erfindungsgemäßen Verfahrens veranschaulichen.

### Beispiel 1

### A. Anreicherung von 9(Z)-C₁₅-Triphenylphosphoniumsalz

4,8 Liter einer Mutterlauge von der technischen Herstellung von 3-Methyl-5-(2,6,6-trimethyl-1-cyclohexen-1-yl)-2,4-pentadienyl-triphenylphosphoniumhydrogensulfat (C₁₅-Triphenylphosphoniumsulfat) in Heptan/Isopropanol wurden mit 480 ml Wasser versetzt und intensiv durchmischt. Nach Abtrennung der organischen Phase wurde die wäßrige Lösung in einem Rotationsverdampfer bei 70°C vollständig eingeengt, wobei portionsweise etwa 3 Liter Isopropanol zunächst zur schonenderen Entfernung des Wassers und letztlich zum Ausfällen von 9(E)-C₁₅-Triphenylphosphoniumhydrogensulfat zugegeben wurde.

Man erhielt 523 g einer etwa 50 gew.-%igen Lösung von C₁₅-Triphenylphosphoniumhydrogensulfat in der nach HPLC-Analyse das 9(Z)-Isomere im Verhältnis 1,5:1 überwog.

Anschließend impfte man die Lösung mit 9(E)-C₁₅-Triphenylphosphoniumhydrogensulfat an, ließ 16 Stunden bei 5°C stehen und filtrierte dann das auskristallisierte 9(E)-C₁₅-Salz ab. Es verblieben 336 g Mutterlauge, die noch etwa 2 % Wasser und das C₁₅-Triphenylphosphoniumhydrogensulfat in einem Verhältnis von 8,4:1 enthielt und bei 5°C ohne nennenswerte Zersetzung lagerfähig ist.

### B. Wittig-Olefinierung mit β-Apo-12'-carotinal

Ein Gemisch aus etwa 120 g (ca. 1 mol) der gemäß Beispiel 1A hergestellten C₁₅-Triphenylphosphoniumhydrogensulfat-Lösung, 158 ml Wasser, 180 ml n-Hexan und 36 g (0,1 mol) β-Apo-12'-carotinal (C₂₅-Al) wurde bei 20°C vorgelegt und dazu innerhalb von 20 Minuten 56,3 g (0,31 mol) einer 30 gew.-%igen methanolischen Lösung von Natriummethylat zugetropft. Man rührte noch 1 Stunde bei 50°C nach, setzte dann 240 ml Methanol hinzu und trennte die Unterphase ab. Die Heptanphase wurde bei 50°C mit 110 ml eines 60 gew.-%igen wäßrigen Methanols gewaschen und am Rotationsverdampfer eingeengt. Man erhielt 61 g eines Rohproduktes, aus welchem durch Filtration über Kieselgel und Waschen mit Cyclohexan/Diisopropylether (9:1) noch enthaltenes C₂₅-Al abgetrennt wurde. Nach Eindampfen des β-Carotin enthaltenden Filtrats am Rotationsverdampfer erhielt man 48,1 g (entsprechen 86,1 % der Theorie) eines β-Carotin-Isomerengemisches, welches gemäß HPLC die folgende Zusammensetzung aufwies:
1,4 % 13(Z)-β-Carotin, 54,6 % 9(Z)-β-Carotin, 11 % all(E)-β-Carotin, 33 % 9(Z), 11(Z)-β-Carotin und andere Isomere.

### C. Isomerisierung

Eine Lösung des gemäß Beispiel 1B hergestellten β-Carotin-Isomerengemisches in 400 ml n-Hexan wurde 2 Stunden unter Rückfluß auf etwa 70°C erwärmt und dann am Rotationsverdampfer eingeengt. Das als viskoses Öl anfallende β-Carotin-Isomerengemisch wies gemäß HPLC folgende Zusammensetzung auf:
2,6 % 13(Z)-β-Carotin, 68,9 % 9(Z)-β-Carotin, 11,3 % all(E)-β-Carotin und 17,2 % andere Isomere.

### Beispiel 2

Ein zweiphasiges Gemisch bestehend aus 120 g (ca. 0,1 mol) der gemäß Beispiel 1A hergestellten 9(Z)-C₁₅-Triphenylphosphoniumsalzlösung und einer Lösung von 18 g (0,11 mol) 2,6-Dimethylocta-2,4,6-trien-1,8-dial in 220 ml Dichlormethan wurde bei ca. 30°C bis zur Sättigung mit Ammoniak begast. Anschließend wurden die Phasen getrennt, die Dichlormethanphase am Rotationsverdampfer eingeengt und der dabei erhaltene Rückstand in einem Gemisch aus 150 ml Heptan und 100 ml Methanol unter Erwärmen auf 50°C gelöst. Nach Zugabe von 65 ml Wasser trennte man die Unterphase ab. Die Oberphase enthielt gemäß HPLC-Analyse ein (E,Z)-Gemisch von β-Apo-12'-carotinal enthaltend etwa 56 9(Z)-und etwa 30 % (9Z, 11Z)-β-Apo-12'-carotinal.

Die Heptanphase wurde anschließend mit einer Lösung von 62 g eines etwa 90 %igen β-Ionylidenethyl-triphenylphosphoniumhydrogensulfats in 100 ml Methanol und danach bei ca. 30°C tropfenweise mit 45,5 g (0,25 mol) einer 30 gew.-%igen methanolischen Natriummethylat-Lösung versetzt.

Nach 30 min setzte man 120 ml Wasser zu, trennte die Unterphase bei 50°C ab und wusch die Oberphase zweimal mit je 120 ml eines 60 %igen wäßrigen Methanols. Die Heptanphase wurde am Rotationsverdampfer eingeengt (50 mbar, 50°C Badtemperatur) und ergab 61,5 g (E,Z)-β-Carotin.

Das Rohprodukt wurde wie in Beispiel 1 beschrieben durch Filtration über Kieselgel gereinigt, in 200 ml Heptan aufgenommen und thermisch isomerisiert (1 h bei 80°C), wobei die (11Z)- und (11'Z)-konfigurierten Doppelbindungen in die E-Form überführt wurden. Nach Einengen am Rotationsverdampfer fiel das β-Carotin als viskoses Öl an und wies gemäß HPLC-Analyse folgende Zusammensetzung auf: 4,4 % 13(Z)-β-Carotin, 66 % 9(Z)-β-Carotin, 9,6 % all E-β-Carotin und ca. 20 andere Isomere des β-Carotins.

## Patentansprüche

1. Verfahren zur Herstellung von β-Carotinpräparaten mit Anteilen von 60 bis 80 % (HPLC-Flächen-%) an 9(Z)-β-Carotin der Formel I 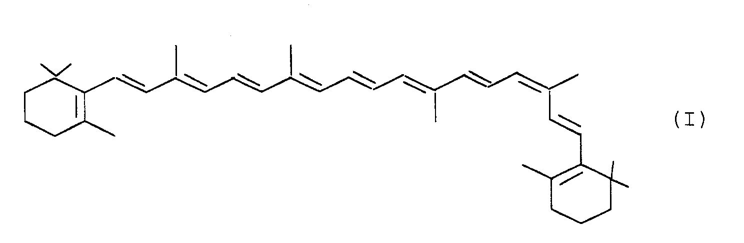 ausgehend von Mutterlaugen der technischen Herstellung von β-Ionylidenethyl-triarylphosphoniumsalzen (C₁₅-Triarylphosphoniumsalzen), dadurch gekennzeichnet, daß man
A. in den aus den Mutterlaugen durch Extraktion mit Wasser und Einengen der wäßrigen Phase isolierten C₁₅-Triarylphosphoniumsalzen den Anteil an 9(Z)-C₁₅-Triarylphosphoniumsalzen dadurch anreichert, daß man das ölige C₁₅-Triarylphosphoniumsalzgemisch in einem niederen Alkanol löst und das beim Abkühlen auskristallisierende all-(E)-C₁₅-Triarylphosphoniumsalz abtrennt,
B. das erhaltene, an dem 9(Z)-Isomeren der allgemeinen Formel II 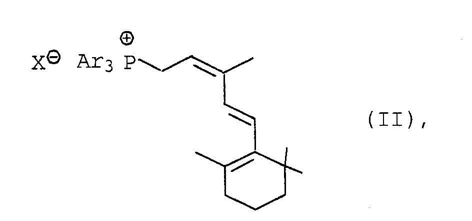 in der X für Halogen oder den Rest (HSO₄)⁻ und Ar für ggf. substituiertes Phenyl steht, angereicherte C₁₅-Triarylphosphoniumsalz in Gegenwart einer Base in einem für Wittig-Reaktionen üblichen Lösungsmittel
a) entweder direkt mit all-(E)-β-Apo-12'-carotinal der Formel III 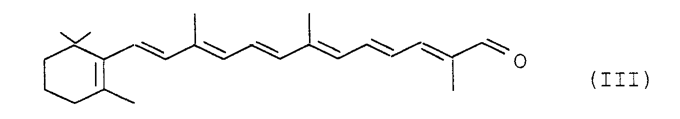 oder aber
b) zunächst mit dem symmetrischen C₁₀-Dialdehyd der Formel IV 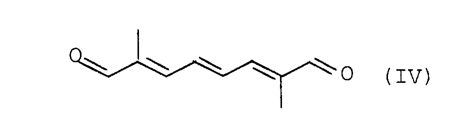 umsetzt und dann das dabei erhaltene 9(Z)-β-Apo-12'-carotinal der Formel V 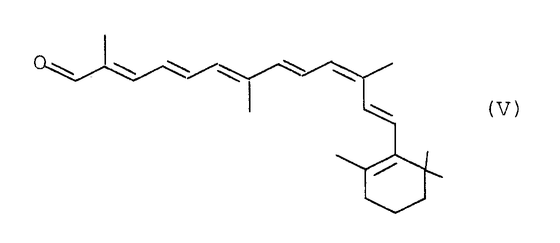 in Gegenwart einer Base in einem für Wittig-Reaktionen üblichen Lösungsmittel mit einem 9-(E)-β-Ionylidenethyl-triarylphosphoniumsalz der allgemeinen Formel VI 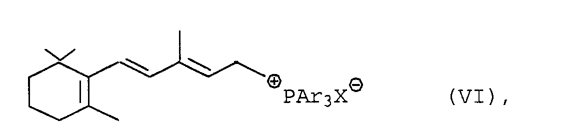 in der X^{⊖} und Ar die oben angegebenen Bedeutung haben, umsetzt und
C. das erhaltene und auf übliche Weise isolierte β-Carotin zwecks Isomerisierung der gebildeten (Z)-konfigurierten Doppelbindung in 11- bzw. 11'-Stellung von β-Carotin einer thermischen Isomerisierung unterwirft.

2. Verfahren gemäß Anspruch 1, dadurch gekennzeichnet, daß man in Stufe B als Base ein organisches Amin, Ammoniak, ein Alkalicarbonat oder ein Alkalialkoholat verwendet.

3. Verfahren gemäß Anspruch 1, dadurch gekennzeichnet, daß man das erhaltene und auf übliche Weise isolierte β-Carotin zwecks Isomerisierung der gebildeten (Z)-konfigurierten Doppelbindung in 11- bzw. 11'-Stellung von β-Carotin in einem inerten Lösungsmittel etwa 1 bis 2 Stunden auf Temperaturen von 70 bis 80°C erwärmt.

4. Verfahren gemäß Anspruch 3, dadurch gekennzeichnet, daß man als inertes Lösungsmittel Heptan oder Isobutanol verwendet.

5. Verfahren gemäß Anspruch 1, dadurch gekennzeichnet, daß man das in Reaktionsstufe Bb erhaltene und auf übliche Weise isolierte rohe β-Apo-12'-carotinal der Formel V zwecks Isomerisierung der gebildeten (Z)-konfigurierten Doppelbindung in 11- bzw. 11'-Stellung von β-Carotin in einem inerten Lösungsmittel etwa 1 bis 2 Stunden auf Temperaturen von 70°C bis 80°C erwärmt.

6. Verfahren gemäß Anspruch 5, dadurch gekennzeichnet, daß man als inertes Lösungsmittel Heptan oder Isobutanol verwendet.

7. Verfahren gemäß Anspruch 1, dadurch gekennzeichnet, daß man im Reaktionsschritt A den Anteil an dem 9-(Z)-C₁₅-Triarylphosphoniumsalz aus den Mutterlaugen der C₁₅-Triarylphosphoniumsalz-Herstellung dadurch anreichert, daß man
a) die Mutterlauge mit Wasser versetzt und die organische Phase abtrennt,
b) die wäßrige Phase unter schonenden Bedingungen einengt und dabei einen Lösungsmittelaustausch von Wasser zu Isopropanol vornimmt, und
c) das durch Abkühlen auskristallisierende all-(E)-C₁₅-Triarylphosphoniumsalz abtrennt.

## Claims

1. A process for preparing β-carotene products with proportions of from 60 to 80% (HPLC % areas) of 9(Z)-β-carotene of the formula I 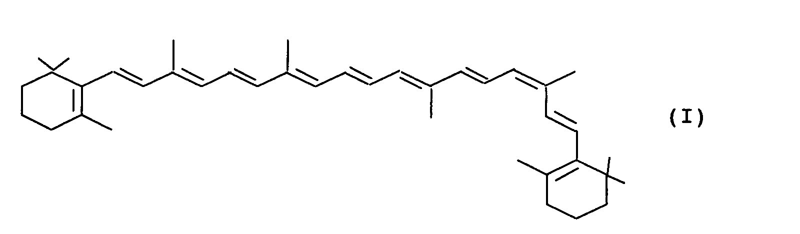 starting from mother liquors from the industrial preparation of β-ionylideneethyltriarylphosphonium salts (C₁₅-triarylphosphonium salts), which comprises
A. the proportion of 9(Z)-C₁₅-triarylphosphonium salts in the C₁₅-triarylphosphonium salts isolated from the mother liquors by extraction with water and concentration of the aqueous phase being increased by dissolving the oily C₁₅-triarylphosphonium salt mixture in a lower alkanol and removing the all-(E)-C₁₅-triarylphosphonium salt which crystallizes out on cooling,
B. the resulting C₁₅-triarylphosphonium salt which is enriched in the 9(Z)-isomer of the general formula II 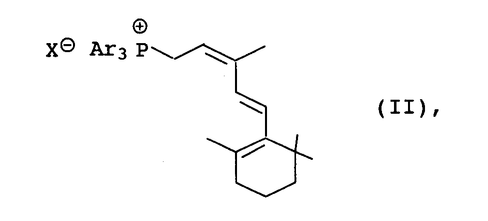 where X is halogen or HSO₄⁻ and Ar is unsubstituted or substituted phenyl being reacted in the presence of a base in a solvent customary for Wittig reactions
a) either directly with all-(E)-β-apo-12'-carotenal of the formula III 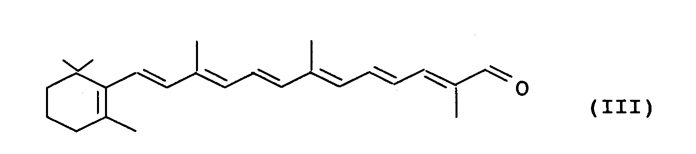 or else
b) in the first place with the symmetrical C₁₀-dialdehyde of the formula IV 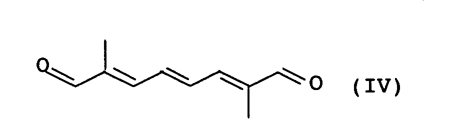 and then the resulting 9(Z)-β-apo-12'-carotenal of the formula V 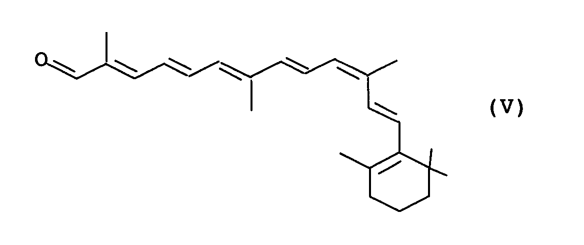 being reacted in the presence of a base in a solvent customary for Wittig reactions with a 9-(E)-β-ionylideneethyltriarylphosphonium salt of the general formula VI 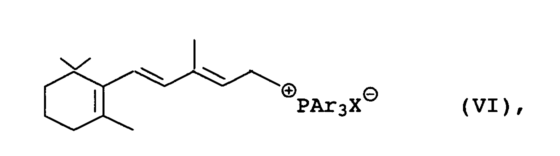 where X^{⊖} and Ar have the abovementioned meanings, and
C. the resulting β-carotene which has been isolated in a conventional way being subjected to a heat treatment to isomerize the double bond with the (Z) configuration formed in the 11 or 11' position of β-carotene.

2. A process as claimed in claim 1, wherein an organic amine, ammonia, an alkali metal carbonate or an alkali metal alcoholate is used as base in stage B.

3. A process as claimed in claim 1, wherein the resulting β-carotene which has been isolated in a conventional way is heated at from 70 to 80°C in an inert solvent for about 1 to 2 hours to isomerize the double bond with the (Z) configuration formed in the 11 or 11' position of β-carotene.

4. A process as claimed in claim 3, wherein heptane or isobutanol is used as inert solvent.

5. A process as claimed in claim 1, wherein the crude β-apo-12'-carotenal of the formula V obtained in stage Bb and isolated in a conventional way is heated at from 70°C to 80°C in an inert solvent for about 1 to 2 hours to isomerize the double bond with the (Z) configuration formed in the 11 or 11' position of β-carotene.

6. A process as claimed in claim 5, wherein heptane or isobutanol is used as inert solvent.

7. A process as claimed in claim 1, wherein the proportion of 9(Z)-C₁₅-triarylphosphonium salt from the mother liquors of the C₁₅-triarylphosphonium salt preparation is increased in step A by
a) adding water to the mother liquor and removing the organic phase,
b) concentrating the aqueous phase under mild conditions while changing the solvent from water to isopropanol, and
c) removing the all-(E)-C₁₅-triarylphosphonium salt which crystallizes out on cooling.

## Revendications

1. Procédé pour la préparation de bêta-carotènes contenant de 60 à 80 % (aire % à la CLHP) de 9(Z)-bêta-carotène de formule I 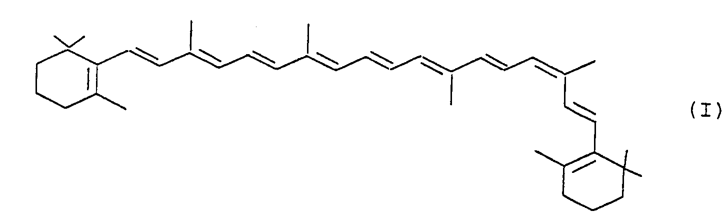 à partir des liqueurs-mères de la fabrication industrielle de sels de bêta-ionylidénéthyltriarylphosphonium (sels de C15-triarylphosphonium), caractérisé par le fait que
A.- dans les sels de C₁₅-triarylphosphonium isolés des liqueur-mères par extraction à l'eau et concentration de la phase aqueuse, on enrichit la fraction de sels de 9(Z)-C₁₅-triarylphosphonium en dissolvant le mélange huileux des sels de C₁₅-triarylphosphonium dans un alcanol inférieur et en séparant le sel de tout-(E)-C₁₅-triarylphosphonium qui cristallise au refroidissement,
B. en faisant réagir le sel de C₁₅-triarylphosphonium ainsi obtenu, enrichi en l'isomère 9(Z) de formule générale II 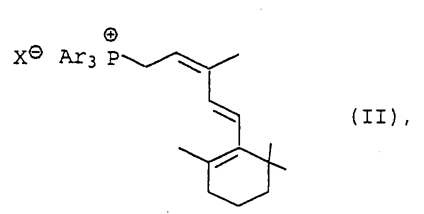 dans laquelle X représente un halogène ou un groupe (HSO4)⁻ et Ar un groupe phényle éventuellement substitué, en présence d'une base et dans un solvant usuel pour les réactions de Wittig
a) soit directement avec le tout-(E)-bêta-apo-12'-caroténal de formule III 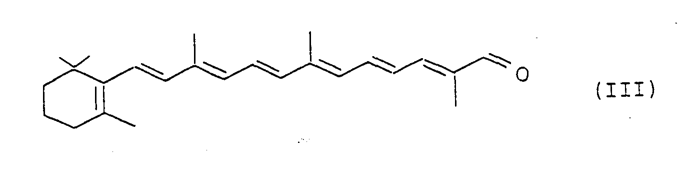
b) soit en faisant réagir d'abord avec le dialdéhyde symétrique en C10 de formule IV 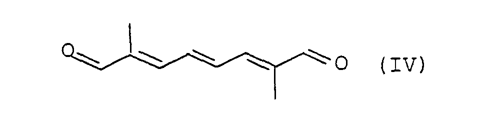 puis en faisant réagir le 9(Z)-bêta-apo-12'-caroténal ainsi obtenu, répondant à la formule V 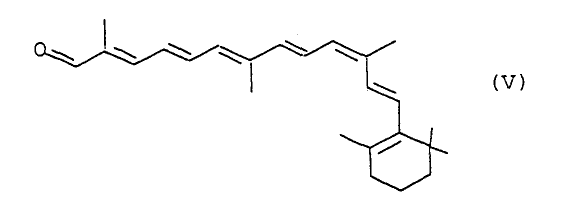 en présence d'une base et dans un solvant usuel pour les réactions de Wittig, avec un sel de 9-(E)-bêta-ionylidénéthyl)triarylphosphonium de formule générale VI 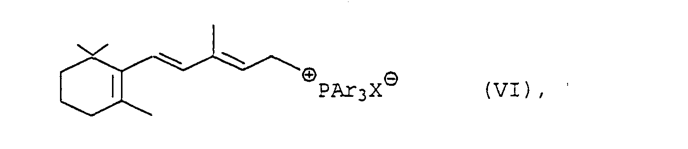 dans laquelle X⁻ et Ar ont les significations indiquées ci-dessus, et
C. en soumettant le bêta-carotène ainsi obtenu et isolé de la manière habituelle à un traitement à la chaleur en vue d'une isomérisation de la double liaison en configuration Z formée dans la position 11 ou la position 11' du bêta-carotène.

2. Procédé selon la revendication 1, caractérisé par le fait que, au stade B, la base utilisée est une amine organique, l'ammoniac, un carbonate alcalin ou un alcoolate alcalin.

3. Procédé selon la revendication 1, caractérisé par le fait que le bêta-carotène obtenu et isolé de la manière habituelle est soumis à un chauffage d'environ 1 à 2 h à des tempréatures de 70 à 80°C dans un solvant inerte en vue d'une isomérisation de la double liaison en configuration 2 formée dans la position 11 ou la position 11' du bêta-carotène.

4. Procédé selon la revendication 3, caractérisé par le fait que le solvant inerte utilisé est l'heptane ou l'isobutanol.

5. Procédé selon la revendication 1, caractérisé par le fait que, au stade de réaction Bb, le bêta-apo-12'-caroténal brut de formule V obtenu et isolé de la manière habituelle est chauffé pendant une durée d'environ 1 à 2 h à des températures de 70 à 80°C dans un solvant inerte en vue d'une isomérisation de la double liaison en configuration 2 formée dans la position 11 ou la position 11' du bêta-carotène.

6. Procédé selon la revendication 5, caractérisé par le fait que le solvant inerte utilisé est l'heptane ou l'isobutanol.

7. Procédé selon la revendication 1, caractérisé par le fait que, au stade de réaction A, on enrichit la fraction de sel de 9-(Z)-C15-triarylphosphonium des liqueur-mères de la fabrication des sels de C15-triarylphosphonium et par le fait que
a) on ajoute de l'eau aux liqueur-(mères et on sépare la phase organique,
b) on concentre la phase aqueuse dans des conditions ménagées et on procède à un échange de solvant, passant de l'eau à l'isopropanol, et
c) on sépare le sel de tout-(E)-C15-triarylphsophonium qui cristallise au refroidissement.
